# EUROPEAN PATENT SPECIFICATION

(11) **EP 4 093 326 B1**
(45) Date of publication and mention of the grant of the patent: **29.04.2026**
(21) Application number: 21702191.4
(22) Date of filing: 22.01.2021
(51) Int. Cl.: A61F 2/07

(54) **ENDOPROSTHESIS AND METHOD OF MANUFACTURING AN ENDOPROSTHESIS**
ENDOPROTHESE UND VERFAHREN ZUR HERSTELLUNG EINER ENDOPROTHESE
ENDOPROTHÈSE ET PROCÉDÉ DE FABRICATION D'UNE ENDOPROTHÈSE

(30) Priority: 23.01.2020 EP 20315008
(43) Date of publication of application: 30.11.2022
(73) Proprietor: Kardiozis SAS, 13100 Aix-en-Provence (FR)
(72) Inventor: VANDAELE-FENOUIL, Nathalie, 13100 Aix-en-Provence (FR); CHAPUT, Oriane, 13100 Aix-en-Provence (FR); BURG, Brian, 13100 Aix-en-Provence (FR)
(74) Representative: Hepp Wenger Ryffel AG
(86) International application number: PCT/EP2021/051401
(87) International publication number: WO 2021/148578

(56) References cited:
- EP-A1- 2 769 699
- EP-A1- 2 962 702
- WO-A1-2004/002365
- WO-A1-99/37242

## Description

The present invention relates to an endoprosthesis and a method of manufacturing an endoprosthesis according to the independent claims.

Endoprostheses, in particular vascular and heart stents, are used to support blood vessels in the human body. For example, occlusions or aneurysms can be treated by placing such an endoprosthesis at the respective treatment site. In the treatment of an occlusion, the endoprosthesis keeps the vessel open for unhindered blood flow. In the case of an aneurysm, the endoprosthesis can prevent circulation of blood into the aneurysm and thus lower the risk of a thrombus, rupture or further growth of the aneurysm.

It is known in the prior art to use thrombogenic elements on endoprostheses. For example, WO 2013/182614 A1 discloses an endoprosthesis with thrombogenic elements that extend away from a body of the endoprosthesis and promote thrombosis. This allows for the occlusion of an aneurysm to enhance the above-mentioned treatment effect.

WO 2006/0166167 discloses fibers arranged on an endovascular prosthesis.

WO 2019/122944 discloses the attachment of a strip of fabric on with fibers on a stent graft.

DE 195 31 659 discloses a stent wire which comprises fibers. The fibers are attached to the wire structure by being held between two wires that form a spiral. In particular, the fibers of DE 195 31 659 are arranged on the wire used to produce the stent structure. They cannot be attached to the stent post-production.

EP 2 769 699 discloses stent-grafts having a continuous anchoring machine stitching and methods of attachment of a stent to the graft material.

WO 2004/002365 discloses a fastening for fixing an exposed stent to a graft material. At least two spaced-apart fastenings are used, each fastening has at least one turn and preferably two turns of an elongate flexible fiber through the graft material and around a portion of the stent.

Currently known methods hence do not provide a simple way of post-production arrangement of thrombogenic elements on an endoprosthesis. Fixation and attachment of thrombogenic elements is usually cumbersome and difficult, and not typically versatile. In addition, known endoprosthesis are limited to generic thrombus generation means that are not adapted to patient-specific needs.

Thus, the object of the present invention is to overcome the drawbacks of the prior art, in particular to provide an alternative mechanism to attach a fiber to an endoprosthesis in a simple, cheap and reliable way.

This and other objects are achieved by the endoprosthesis and the methods according to the characterizing portion of the independent claims.

The endoprosthesis according to the invention may in particular be a vascular or a heart stent. The endoprosthesis comprises a graft, a stent structure, a fiber, and a suture. The suture fixedly attaches the stent structure to the graft. The fiber is attached to the endoprosthesis via the suture. Such an endoprosthesis is particularly advantageous because the fiber attachment is completely independent from the selection of graft and stent structure. Any type of fiber may be attached to any type the endoprosthesis, regardless of the selection of the stent structure and graft. It also allows for the stent and/or the graft to be pretreated by any treatment, such as heat, plasma, pressure, or radiation, that may otherwise damage the fibers or an attachment mechanism for the fibers.

The fiber is attached to the stent structure and to the graft via the suture.

The fiber is a thrombogenic fiber. The thrombogenic fiber extends, at least partly, away from the surface of the graft and/or stent structure. The thrombogenic fiber has a free portion, for example a free end, that extends a certain distance away (e.g. outwardly away) from the surface of the graft and/or stent structure. The thrombogenic fiber may, in use, promote thrombosis in a region around or adjacent the endoprosthesis, for example, thrombosis in an aneurysm as explained above. For example, the thrombogenic fiber may have a length of at least 2mm, optionally between 2mm and 50mm. Additionally or alternatively, for example, the thrombogenic fiber may have a free portion having a length of, and/or that can extend a distance away from a surface of the graft and/or stent structure by, at least 2mm, optionally between 2mm and 50mm.

The suture may retain attachment of the fiber to the stent structure and/or graft after implantation of the endoprosthesis.

This can keep the thrombogenic effect in the intended placement within the anatomy.

The fiber **(e.g.** thrombogenic fiber) may be distinct from the graft. The fiber **(e.g.** thrombogenic fiber) is distinct from the suture.

The fiber may comprise or consist of any suitable material for the desired application, for example Dacron, polyethylene, polypropylene, polytetrafluoroethylene, a polyamide (for example, Nylon), polyglycolic-lactic acid or another biocompatible polymer with a thrombogenic effect. However, metals and ceramics are also conceivable as fiber materials.

The suture is preferably a surgical thread known in the art. Alternatively, the suture may also be substantially identical to the fiber, or made of another material.

The suture and the fiber also may be made of the same material and configured integrally.

Preferably, the suture has a knot. The knot may in particular form the suture into a substantially closed loop holding together the stent structure and the graft. This allows for a high degree of flexibility in the attachment of stent and graft, but also a firm attachment of fiber to the endoprosthesis. A substantially closed loop with a knot may be arranged at any desired position on the endoprosthesis.

The substantially closed loops may be arranged as separate, discrete loops that are not connected to other loops.

Preferably, the knot connects the suture to the fiber. The fiber may be configured as a separate element attached by the knot. Alternatively, in particular if the fiber and the suture are made of the same material and/or formed integrally, the free ends of the suture extending away from the knot may be the fibers.

The fiber may form a loop around the suture. The loop may be a substantially closed loop, or a loop that is not closed. The loop may be fixed by a knot, an adhesive, a mechanical fixation, or another fixation mechanism. Alternatively, the loop may only loosely loop around the suture, as a closed loop or as a loop that is not closed.

Preferably, the suture forms a continuous stitch, in particular a buttonhole stitch. The continuous stitch may particularly preferably hold the stent structure and the graft together. A continuous stitch is particularly advantageous because it provides secure attachment and can be arranged in a continuous process. In addition, it provides further flexibility to attach fibers at different longitudinal positions, along the stent structure.

Particularly preferably, the fiber is attached with a conventional continuous stitch as known in the art. This allows for a particularly easy process to attach the graft to the stent structure. The fiber may be attached in the same process as the attachment of stent structure and graft, i.e. while arranging the suture. Alternatively, the fiber may also be attached to the suture after the attachment of stent structure and graft.

Preferably, endoprosthesis comprises at least two fibers, wherein the at least two fibers are connected via a common additional support string which is connected to the suture. A support string provides for a particularly efficient attachment process because multiple fibers can be attached at the string in one process step.

Preferably, the fiber comprises a knot connecting the fiber to the suture. A knot comprised by the fiber enables a particularly flexible attachment of the fiber to the suture and substantially any position on the suture.

The invention further relates to a method of manufacturing an endoprosthesis. The method is performed to manufacture an endoprosthesis as described herein. The method comprises a first step of providing a stent structure, a graft, and a suture. The stent structure is attached to the graft via the suture. At least one fiber (e.g. thrombogenic fiber) is attached to the stent structure and to the graft via the suture, in particular directly to the suture. In particular, the fiber may be attached via at least one of a loop, a knot, and a support string comprised by the fiber and/or the suture. The person skilled in the art will understand that the method may encompass steps of arranging the fiber, the suture, the stent structure and/or the graft as described herein.

In the following, the invention is described in detail with reference to the following figures, showing:
- Fig. 1:: a detail of a first embodiment of an endoprosthesis in a cross-sectional view perpendicular to an axis,
- Fig. 2:: a detail of a second embodiment of an endoprosthesis in a cross-sectional view perpendicular to an axis,
- Fig. 3:: a detail of a third embodiment of an endoprosthesis in a cross-sectional view perpendicular to an axis,
- Fig. 4:: a detail of a fourth embodiment of an endoprosthesis in a cross-sectional view perpendicular to an axis,
- Fig. 5:: a detail of a fifth embodiment of an endoprosthesis in a view along an axis,
- Fig. 6:: a detail of a sixth embodiment of an endoprosthesis in a cross-sectional view along an axis,
- Fig. 7a-7b:: a detail of a seventh embodiment of an endoprosthesis in a cross-sectional view perpendicular to and along an axis
- Fig. 8:: an exemplary endoprosthesis according to the invention.

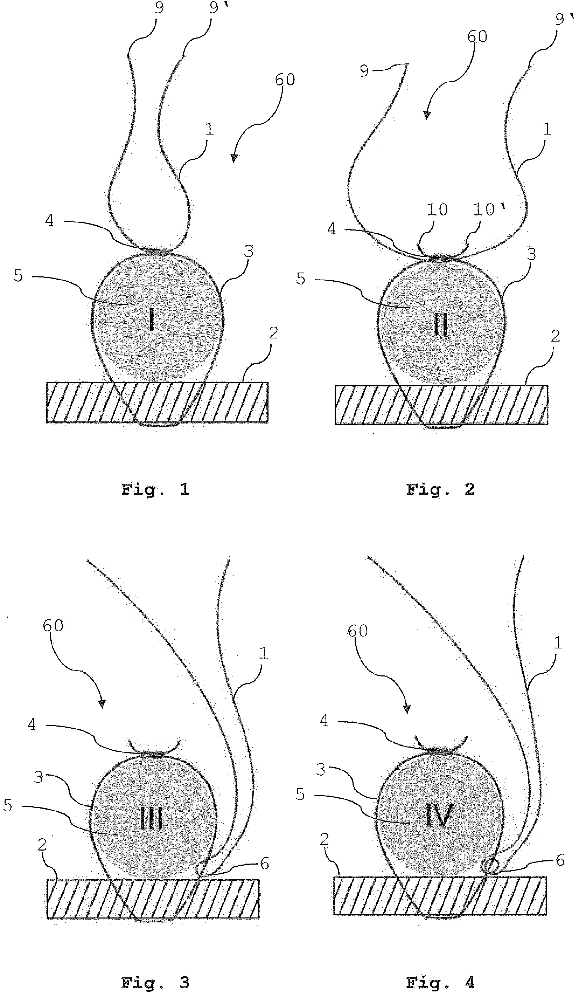

Fig. 1 shows a cross-sectional view of a portion of an endoprosthesis 60 in a plane perpendicular to a longitudinal axis of a strut of the stent structure 5. The endoprosthesis comprises a graft 2 made of a biocompatible polymer material, for example polyethylene terephthalate, and a stent structure 5 formed of struts made of a Nitinol alloy. A suture 3, which in the present case is a Dacron (i.e. polyethylene terephthalate) fiber, is arranged around the stent structure 5 to form a substantially closed loop that penetrates the graft 2. It therefore holds the stent structure 5 and the graft 2 together in a fixed manner. The suture 3 further comprises a knot 4 that forms the substantially closed loop. The knot 4 further connects the suture 3 to two thrombogenic fibers 1 having a free end 9,9' each. In the present embodiment, the fibers 1 are formed integrally with the suture 3, meaning that the fibers 1 consist of the free ends 9,9' of the suture. Furthermore, in the present embodiment, the suture 3 forms exactly one loop around the stent structure 5. There may be a plurality of sutures and loops along the axis of the strut. Each loop is formed by one knot 4 which connects the suture 3 to the fiber.

Fig. 2 shows an alternative embodiment of the endoprosthesis 60. The present embodiment is similar to the embodiment of Fig. 1. Here, the suture 3 and the fiber 1 are configured as separate elements. The suture 3 in this embodiment is a conventional surgical thread known in the art and extends around the stent structure 5. The suture 3 comprises a knot that forms the substantially closed loop. The free ends 10,10' of the suture do not substantially extend away from the knot. They do not have a substantial thrombogenic effect. The fiber 1 extends through the knot 4 and is attached to the suture 3 via the knot 4. Two free ends 9,9' of the fiber 1 extend away from the knot. The fiber 1 is made of polyethylene, but may alternatively comprise or consist of polyethylene terephthalate, polyamide, or polyglycolic-lactic acid. The fiber 1 may additionally be functionalized on its surface to increase its thrombogenicity and to increase friction within the knot 4 to achieve a secure attachment.

Fig. 3 shows an alternative embodiment of the endoprosthesis 60 which is similar to the embodiment of Fig. 2. The fiber 1, however, is not attached to the knot 4 of the suture. Instead, the fiber 1 extends through the substantially closed loop formed by the suture 3 and forms a loop 6 around the suture 3. The loop 6 in the present embodiment is not closed and is only held in position by friction between the suture 3 and the fiber 1. This method of attachment is particularly advantageous because it is easily adaptable and typically faster than conventional methods. The fiber 1 can easily be removed, moved to a different position, or changed in its length. While not shown here, it would of course be possible to additionally use an adhesive, a heat treatment such as welding, or another additional attachment mechanism to create a more secure attachment.

Fig. 4 shows yet an alternative embodiment of the endoprosthesis 60. The shown endoprosthesis 60 is similar to the embodiment of Fig. 3. However, the fiber 1 forms a substantially closed loop 6 around the suture for a more secure attachment.

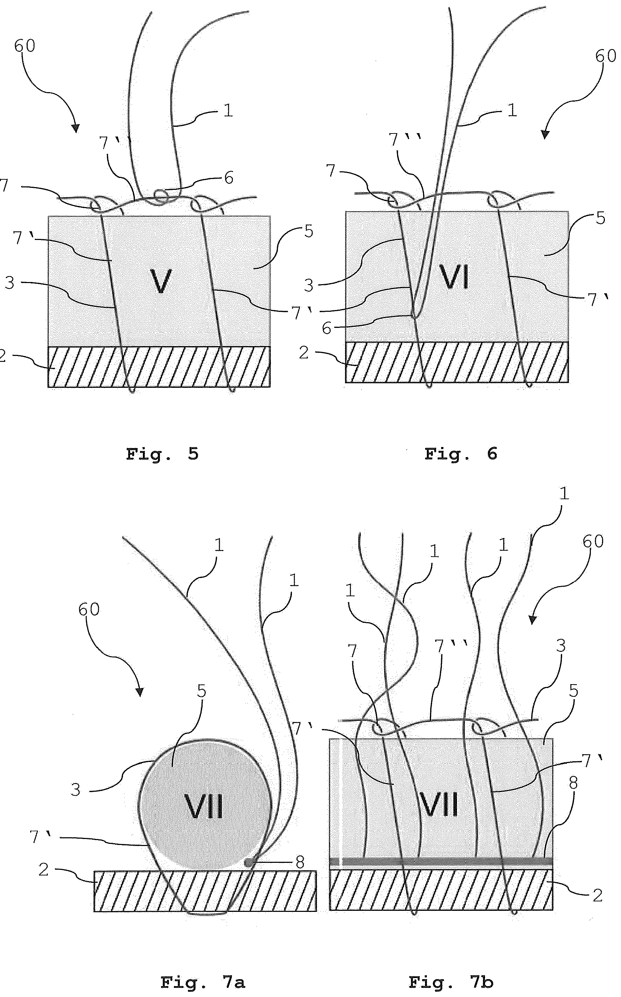

Fig. 5 shows a cross-sectional view of an endoprosthesis 60 in a plane that is arranged parallel to the stent structure 5. The suture 3 extends as a continuous loop-structure around and along the stent structure 5 and the graft 2 such as to fix both together. The suture 3 is configured as buttonhole stitch 7,7',7'' and thus comprises a loop portion 7', a knot portion 7, and a loop-connecting portion 7". The loops 7' of the buttonhole stitch extend around the graft 2 and the stent structure 5 and hold them together. The knot portions 7 form the loops 7' which are connected to one another by the loop-connecting portions 7". The suture 3 is made of conventional surgical thread without a substantial thrombogenic effect. The thrombogenic fiber 1 is made of polyamide, but may alternatively comprise or consist of polyethylene terephthalate, polypropylene, or polyglycolic-lactic acid, and is attached to the loop-connecting portion 7'' of the buttonhole stitch. The fiber 1 forms a substantially closed loop 6 around the loop-connecting portions 7" and is held there by friction between the suture 3 and the fiber 1. While not shown here, it would of course be possible to additionally use an adhesive, a heat treatment such as welding, or another additional attachment mechanism to create a more secure attachment. By contrast, if a high degree of flexibility is desired, the fiber 1 could also be attached by a loop 6 that is not closed in a similar fashion as shown in Fig. 3.

Fig. 6 shows an alternative embodiment of the endoprosthesis 60 similar to the embodiment shown in Fig. 5. In this embodiment, the fiber 1 forms a loop 6 that is not substantially closed as also shown in Fig. 3. Here the fiber 1 is attached to the suture in the loop 7' of the buttonhole stitch 7,7',7''.

Fig. 7a shows a cross-sectional view of a portion of an endoprosthesis 60 in a plane perpendicular to a longitudinal axis of the stent structure 5. The suture 3 is formed as a buttonhole stich and holds the stent structure 5 and the graft 2 together substantially as shown in Figs. 5 and 6. Because the endoprosthesis 60 is shown in a cross-sectional view, only the first loop 7' of the buttonhole stitch is visible. The fibers 1, only two of which are visible in the shown perspective, are attached to an additional support string 8 that extends in a substantially parallel manner along the stent structure 5 and through the loops 7' of the buttonhole stitch. The support string 8 is therefore fixedly attached to the endoprosthesis 60 via the suture and the support string.

Fig. 7b shows the embodiment of Fig. 7a in a cross-sectional view in a plane that is arranged parallel to the stent structure 5. The support string 8 extends parallel to the stent structure 5 and is arranged within the loops 7' of the buttonhole stitch 7,7',7". The fibers 1 extend away from the graft 2 and the stent structure 5 and are attached to the endoprosthesis 60 via the support string 8 and the suture 3.

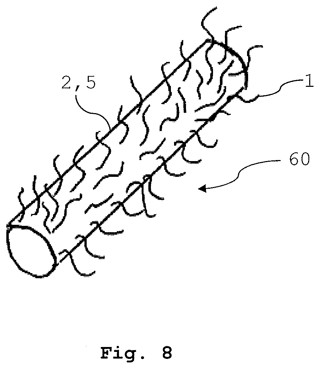

Fig. 8 shows an exemplary endoprosthesis 60 according to the invention. The endoprosthesis 60 comprises a stent formed of a plurality of bars or stents 5 in a manner known in the art and a graft 2 which are attached to one another through a suture (not shown) to which fibers 1 are attached. At least one, and preferably several, fibers 1 are arranged on the endoprosthesis 60. The fibers 1 can be thrombogenic fibers for promoting thrombosis around or adjacent the endoprosthesis 60. The fibers 1 may have a portion (e.g. at least one end and/or or at least one intermediate portion) that is free to extend away from the graft and/or stent, for example, by at least a certain distance. The fibers 1 optionally may be distinct from the graft.

Any of the above-mentioned attachment mechanisms is suitable to attach the fibers 1 to the endoprosthesis 60 (e.g. to the stent and/or graft). The fibers 1 may remain attached by the suture, to the stent and/or to the graft after implantation using these attachment mechanisms. In the present embodiment, all the fibers are attached by the same mechanism. However, it would also be conceivable to attach different fibers 1 with different mechanisms and thus include several of the above-described mechanisms in one endoprosthesis. In particular, fibers with varying lengths between 2 and 50 mm may be attached to the stent and/or graft by the above-described mechanisms. Additionally or alternatively, fibers 1 may be attached to the stent and/or graft by the above-described mechanisms such that a free portion of the fiber may have a length of, and/or be able to extend away from the stent and/or graft by a distance of, between 2 and 50mm. Similarly, the present embodiment shows a plurality of fibers 1 attached on the endoprosthesis 60 with a substantially uniform distribution. The person skilled in the art will understand that this is merely an exemplary embodiment and that any number of fibers 1 could be attached to the endoprosthesis 60, or with any distribution desired for a particular application. The fibers are preferably attached to the endoprosthesis 60 by the method according to the invention.

## Claims

1. An endoprosthesis (60), preferably a vascular or a heart stent, comprising a graft (2), a stent structure (5), a fiber (1) and a suture (3), wherein the suture (3) fixedly attaches the stent structure (5) to the graft (2), wherein the fiber (1) is attached to the stent structure (5) and to the graft (2) via the suture (3) and the fiber (1) is distinct from the suture (3), **characterized in that** the fiber (1) is a thrombogenic fiber having a free portion configured to extend at least partly away from a surface of the stent structure (5) and/or graft (2).

2. Endoprosthesis (60) according to claim 1, wherein the suture (3) has a knot (4), wherein the knot (4) preferably forms the suture (3) into a substantially closed loop holding together the stent structure (5) and the graft (2).

3. Endoprosthesis (60) according to one of the claims 1 or 2, wherein the knot (4) connects the suture (3) to the fiber (1).

4. Endoprosthesis (60) according to one of the preceding claims, wherein the fiber (1) forms a loop (6) around the suture (3).

5. Endoprosthesis (60) according to one of the preceding claims, wherein the suture (3) forms a continuous stitch (7,7',7''), preferably a buttonhole stitch, particularly preferably wherein the continuous stitch (7,7',7'') holds the stent structure (5) and the graft (2) together.

6. Endoprosthesis (60) according to one of the preceding claims, comprising at least two fibers (1), wherein the at least two fibers (1) are connected via a support string (8).

7. Endoprosthesis (60) according to one of the preceding claims, wherein the fiber (1) comprises a knot connecting the fiber (1) to the suture (3).

8. Endoprosthesis (60) according to any preceding claim, wherein the fiber is distinct from the graft.

9. Endoprosthesis (60) according to any preceding claim, wherein the suture (3) is configured to retain attachment of the fiber (1) after implantation of the endoprosthesis (60).

10. A method of manufacturing an endoprosthesis (60) according to one of the preceding claims, comprising the steps of:
- Providing a stent structure (5), a graft (2), and a suture (3);
- Attaching the stent structure (5) to the graft (2) via the suture (3);
- Attaching at least one fiber (1) to the endoprosthesis (60) via the suture (3), preferably to the suture (3), particularly preferably via at least one of a loop (6), knot (4), and support string (8) comprised by the fiber (1) and/or the suture (3), wherein
the step of attaching comprises attaching the fiber (1) to the stent structure (5) and to the graft (2) via the suture (3) and the fiber (1) is distinct from the suture.

## Patentansprüche

1. Eine Endoprothese (60), vorzugsweise ein Gefäß- oder Herzstent, umfassend einen Graft (2), eine Stentstruktur (5), eine Faser (1) und einen Faden (3) umfasst, wobei der Faden (3) die Stentstruktur (5) fest mit dem Graft (2) verbindet, wobei die Faser (1) über den Faden (3) an der Stentstruktur (5) und am Graft (2) befestigt ist und sich die Faser (1) vom Faden (3) unterscheidet, **dadurch gekennzeichnet, dass** die Faser (1) eine thrombogene Faser ist, die einen freien Abschnitt aufweist, der so ausgebildet ist, dass er sich zumindest teilweise von einer Oberfläche der Stentstruktur (5) und/oder des Graft (2) weg erstreckt.

2. Endoprothese (60) nach Anspruch 1, wobei der Faden (3) einen Knoten (4) aufweist, wobei der Knoten (4) den Faden (3) vorzugsweise zu einer im Wesentlichen geschlossenen Schlaufe formt, die die Stentstruktur (5) und den Graft (2) zusammenhält.

3. Endoprothese (60) nach einem der Ansprüche 1 oder 2, wobei der Knoten (4) den Faden (3) mit der Faser (1) verbindet.

4. Endoprothese (60) nach einem der vorstehenden Ansprüche, wobei die Faser (1) eine Schlaufe (6) um den Faden (3) bildet.

5. Endoprothese (60) nach einem der vorstehenden Ansprüche, wobei der Faden (3) einen durchgehenden Stich (7, 7', 7"), vorzugsweise einen Knopflochstich, bildet, insbesondere wobei der durchgehende Stich (7, 7', 7'') die Stentstruktur (5) und den Graft (2) miteinander verbindet.

6. Endoprothese (60) nach einem der vorstehenden Ansprüche, umfassend mindestens zwei Fasern (1), wobei die mindestens zwei Fasern (1) über einen Supportstring (8) verbunden sind.

7. Endoprothese (60) nach einem der vorstehenden Ansprüche, wobei die Faser (1) einen Knoten aufweist, der die Faser (1) mit dem Faden (3) verbindet.

8. Endoprothese (60) nach einem der vorstehenden Ansprüche, wobei sich die Faser vom Graft unterscheidet.

9. Endoprothese (60) nach einem der vorstehenden Ansprüche, wobei der Faden (3) so ausgebildet ist, dass er die Befestigung der Faser (1) nach der Implantation der Endoprothese (60) aufrechterhält.

10. Verfahren zur Herstellung einer Endoprothese (60) gemäß einem der vorstehenden Ansprüche, umfassend die folgenden Schritte:
- Bereitstellung einer Stentstruktur (5), eines Grafts (2) und eines Fadens (3);
- Befestigung der Stentstruktur (5) am Graft (2) mittels des Fadens (3);
- - Befestigen mindestens einer Faser (1) an der Endoprothese (60) mittels des Fadens (3), vorzugsweise am Faden (3), besonders bevorzugt mittels mindestens einer Schlaufe (6), eines Knotens (4) oder eines Supportstrings (8), die aus der Faser (1) und/oder dem Faden (3) bestehen, wobei
der Befestigungsschritt das Befestigen der Faser (1) an der Stentstruktur (5) und am Graft (2) mittels des Fadens (3) umfasst, wobei sich die Faser (1) vom Faden unterscheidet.

## Revendications

1. Endoprothèse (60), de préférence un stent vasculaire ou cardiaque, comprenant un greffon (2), une structure de stent (5), une fibre (1) et une suture (3), dans laquelle la suture (3) fixe solidement la structure de stent (5) au greffon (2), dans laquelle la fibre (1) est fixée à la structure de stent (5) et au greffon (2) par l'intermédiaire de la suture (3) et la fibre (1) est distincte de la suture (3), **caractérisé en ce que** la fibre (1) est une fibre thrombogène comportant une partie libre configurée pour s'étendre au moins partiellement à l'écart d'une surface de la structure de stent (5) et/ou du greffon (2).

2. Endoprothèse (60) selon la revendication 1, dans laquelle la suture (3) comporte un nœud (4), le nœud (4) formant de préférence avec la suture (3) une boucle sensiblement fermée qui maintient ensemble la structure de stent (5) et le greffon (2).

3. Endoprothèse (60) selon l'une des revendications 1 ou 2, dans laquelle le nœud (4) relie la suture (3) à la fibre (1).

4. Endoprothèse (60) selon l'une des revendications précédentes, dans laquelle la fibre (1) forme une boucle (6) autour de la suture (3).

5. Endoprothèse (60) selon l'une des revendications précédentes, dans laquelle la suture (3) forme un point continu (7, 7', 7"), de préférence un point de feston, et plus particulièrement dans laquelle le point continu (7, 7', 7") maintient ensemble la structure de stent (5) et le greffon (2).

6. Endoprothèse (60) selon l'une des revendications précédentes, comprenant au moins deux fibres (1), dans laquelle lesdites au moins deux fibres (1) sont reliées par un fil de support (8).

7. Endoprothèse (60) selon l'une des revendications précédentes, dans laquelle la fibre (1) comporte un nœud reliant la fibre (1) à la suture (3).

8. Endoprothèse (60) selon l'une quelconque des revendications précédentes, dans laquelle la fibre est distincte du greffon.

9. Endoprothèse (60) selon l'une quelconque des revendications précédentes, dans laquelle la suture (3) est conçue pour maintenir la fixation de la fibre (1) après l'implantation de l'endoprothèse (60).

10. Procédé de fabrication d'une endoprothèse (60) selon l'une des revendications précédentes, comprenant les étapes consistant à:
- Comprend une structure de stent (5), un greffon (2) et une suture (3);
- Fixation de la structure de stent (5) au greffon (2) à l'aide de la suture (3);
- Fixer au moins une fibre (1) à l'endoprothèse (60) au moyen de la suture (3), de préférence à la suture (3), et de manière particulièrement préférée au moyen d'au moins un élément parmi une boucle (6), un nœud (4) et un fil de soutien (8) formé par la fibre (1) et/ou la suture (3), dans lequel
l'étape de fixation comprend la fixation de la fibre (1) à la structure de stent (5) et au greffon (2) au moyen de la suture (3), la fibre (1) étant distincte de la suture.
